# EUROPÄISCHE PATENTSCHRIFT

(11) **EP 2 763 660 B1**
(45) Veröffentlichungstag und Bekanntmachung des Hinweises auf die Patenterteilung: **05.09.2018**
(21) Anmeldenummer: 12777860.3
(22) Anmeldetag: 05.10.2012
(51) Int. Cl.: A61K 9/00, A61K 9/14, A61K 47/34

(54) **VERFAHREN ZUR HERSTELLUNG EINER HOMOGENEN PULVERMISCHUNG UND VERFAHREN ZUR HERSTELLUNG EINES IMPLANTATS SOWIE IMPLANTAT**
METHOD FOR PRODUCING A HOMOGENEOUS POWDER MIXTURE AND METHOD FOR PRODUCING AN IMPLANT AND IMPLANT
PROCÉDÉ DE PRODUCTION D'UN MÉLANGE PULVÉRULENT HOMOGÈNE, PROCÉDÉ DE PRODUCTION D'UN IMPLANT ET IMPLANT

(30) Priorität: 05.10.2011 DE 102011114864
(43) Veröffentlichungstag der Anmeldung: 13.08.2014
(73) Patentinhaber: Luye Pharma AG, 83714 Miesbach (DE)
(72) Erfinder: WERNER, Eva, 83623 Ascholding (DE); SPILGIES, Heiko, 81373 München (DE); PIOTROWSKI, Holger, 83714 Miesbach (DE)
(74) Vertreter: Forstmeyer, Dietmar
(86) Internationale Anmeldenummer: PCT/EP2012/004180
(87) Internationale Veröffentlichungsnummer: WO 2013/050169

(56) Entgegenhaltungen:
- EP-A1- 1 219 298
- EP-A2- 0 052 510
- WO-A1-00/33809
- WO-A1-96/24336
- WO-A1-03/022297
- WO-A1-2005/070332
- WO-A2-2008/039488
- US-A- 4 419 340

## Beschreibung

Die vorliegende Erfindung betrifft ein Verfahren zur Herstellung einer homogenen Pulvermischung aus mindestens einem Polymermaterial und mindestens einem aktiven Bestandteil durch Homogenisieren in Suspension, ein Verfahren zur Herstellung eines Implantats durch Weiterverarbeitung dieser homogenen Pulvermischung sowie ein so hergestelltes Implantat.

### Hintergrund der Erfindung

In der modernen Arzneimitteltherapie werden beispielsweise bei der Langzeitbehandlung von Erkrankungen implantierbare Arzneimitteldepots eingesetzt, die auch als Implantate bezeichnet werden. Implantate enthalten häufig einen aktiven Bestandteil wie beispielsweise einen medizinischen Wirkstoff (Arzneimittel bzw. Medikament) in einer Matrix aus einem biologisch abbaubaren Polymermaterial. Die Implantate, die vorzugsweise in Form von zylindrischen Stäbchen aus der Polymer/Wirkstoff-Matrix ausgebildet sind, werden einem Patienten vorzugsweise subkutan injiziert. Beispielsweise durch Hydrolyse der Matrix aus Polymermaterial aufgrund des natürlichen Wasserhaushalts im menschlichen Körper erfolgt ein Abbau des Implantats im Körper des Patienten, wodurch der in der Polymermatrix eingeschlossene oder daran gebundene Wirkstoff kontrolliert *in vivo* freigesetzt und über einen längeren Zeitraum an den Patienten abgegeben wird (Depotfunktion). Der Zeitraum der Abgabe des Medikaments im Körper des Patienten erstreckt sich typischerweise über mehrere Wochen bis Monate, beispielsweise über vier bis sechs Wochen. Implantate werden zum Beispiel bei der Behandlung von Tumorpatienten eingesetzt, wobei das Implantat im Körper des Patienten einen Wirkstoff abgibt, der beispielsweise eine Testosteronsenkung und damit eine Hemmung des Tumorwachstums bewirkt. Ein Beispiel für einen als Implantat verabreichten Wirkstoff ist Goserelin.

In der WO 2005/070332 sind Formulierungen für die Abgabe eines Wirkstoffs über einen längeren Zeitraum offenbart, die eine biologisch abbaubare Matrix und einen Wirkstoff enthalten.

Um eine bestimmbare und verläßliche Abgabe des Wirkstoffs aus dem Implantat zu gewährleisten, ist es besonders wichtig, dass das Implantat homogen ist, d.h. über sein gesamtes Volumen die selben Eigenschaften, z.B. bezüglich biologischer Abbaubarkeit bzw. Löslichkeit, aufweist und der Wirkstoff homogen darin verteilt ist. Die Herstellung von entsprechenden Implantaten bzw. von Polymer/Wirkstoff-Mischungen zur Herstellung von Implantaten erfolgt bisher nach verschiedenen Verfahren, die sich im wesentlichen in die folgenden vier Gruppen A) bis D) unterteilen lassen.

### A) Herstellung durch Schmelzen:

Ein Wirkstoff wird in eine Polymerschmelze eingearbeitet, darin homogen suspendiert bzw. dispergiert. Die so erhaltene Mischung aus Polymer und Wirkstoff wird dann zu einem Implantat weiterverarbeitet, z.B. durch Extrudieren oder Pressen. Nachteilig ist, dass die Anwendung dieses Verfahrens ausschließlich für wärmeunempfindliche Wirkstoffe geeignet ist, die ohne Schaden zu nehmen auf die Schmelztemperatur des verwendeten Polymers erhitzt werden können. Ein Beispiel für die Herstellung durch Schmelzen ist in DE 2 051 580 beschrieben.

### B) Herstellung in Gegenwart von organischen Lösungsmitteln:

Ein Polymer wird in einem geeigneten organischen Lösungsmittel (z.B. Dichlormethan) gelöst und anschließend mit einer wässerigen oder organischen Lösung (z.B. methanolisch) des Wirkstoffs gemischt. Hydrophile Wirkstoffe, optional in wässeriger Lösung, werden dabei in der organischen Polymerlösung suspendiert, dispergiert bzw. emulgiert, beispielsweise als w/o-Emulsion. Danach werden die Lösungsmittel z.B. bei erhöhter Temperatur entfernt ("abgedampft"). Die erhaltene homogenisierte Mischung aus Polymer und Wirkstoff wird dann zu einem Implantat weiterverarbeitet. Nachteilig bei diesem Herstellungsverfahren ist, dass zur vollständigen Verdampfung der verwendeten Lösungsmittel lange Zeiten und/oder erhöhte Temperaturen benötigt werden, so dass beispielsweise die Gefahr einer thermischen Zersetzung der Wirkstoffe besteht, was neben einer Abnahme des Gehalts an aktiven Bestandteilen auch zur Zunahme von Verunreinigungen durch Abbauprodukte führen kann. Ebenfalls nachteilig ist eine lange Trockenzeit, die benötigt wird, um grosse Lösungsmittelmengen vollständig zu entfernen. Ein unvollständig entferntes organisches Lösungsmittel wirkt sich negativ auf die Verträglichkeit aus. Außerdem können durch Lösen und Wiederausfällen eines oder mehrerer Bestandteile der späteren Pulvermischung die Substanzeigenschaften verändert werden, da diese von der jeweiligen Kristallform o.ä. der Bestandteile abhängig sein können. Beispielsweise kann die Aktivität einiger Wirkstoffe, wie z.B. Protein-Wirkstoffe, durch organische Lösungsmittel negativ beeinflusst werden. Beispiele für Herstellungsverfahren in Gegenwart von organischen Lösungsmitteln sind in DE 2 051 580, EP 0 058 481, WO 99/20253, WO 99/38535, WO 00/33809, WO 00/40259, WO 00/66087 und WO 00/76483 beschrieben.

### C) Herstellung in Gegenwart von Wasser:

In Gegenwart von Wasser werden mittels Nassgranulierung Granulate aus Wirkstoff und Polymer hergestellt und anschließend extrudiert, oder es werden Mischungen von Polymeren mit wässerigen Aufschlämmungen von Wirkstoff hergestellt, gefolgt von Trocknen und Extrudieren. Eine Nassgranulierung bedarf aufwendiger Vorbereitungs-, Betriebs- und Reinigungsschritte und ist zeit-, energie- und kostenintensiv. Die Trocknung von feuchten (d.h. wasserhaltigen) Granulaten oder Homogenisaten ist zeitintensiv und langwierig und/oder bedarf erhöhter Temperaturen, die den Wirkstoff zersetzen können. Somit können alle oben beschriebenen Nachteile beobachtet werden. Außerdem können bei langem Kontakt mit Wasser, eventuell unter erhöhter Temperatur, ungewollte Reaktionen stattfinden, die zur Abnahme des Gehalts an aktiven Bestandteilen führen. Derartige Verfahren werden beispielsweise für peptidbasierte Wirkstoffe beschrieben, wie z.B. in WO 98/09613, WO 99/48517 und WO 00/33809.

### D) Lösungsmittelfreie Homogenisierung bei Kryotemperaturen

EP 1 392 248 beschreibt ein Verfahren zur lösungsmittelfreien Homogenisierung von Polymer und Wirkstoff durch Kaltmahlen mit Hilfe einer Kryomühle. Dabei werden Polymer und Wirkstoff als Rohstoffe gemeinsam auf eine in der Kryotechnik angewandte Temperatur herabgekühlt und gemeinsam unterhalb der Glasübergangstemperatur des Polymers homogenisiert, wobei die Schritte des Herabkühlens und Homogenisierens mehrfach hintereinander durchgeführt werden können. Allerdings kann dabei das Produkt mit Abrieb aus der Mühle verunreinigt werden. Da in einer Kryomühle nur begrenzte Mengen verarbeitet werden können, ist dieses Verfahren auf die Verarbeitung im Batch-Betrieb beschränkt, wodurch die Herstellung größerer Chargen mit identischen Eigenschaften erschwert ist. Außerdem ist dieses Verfahren auch zeit-, energie- und kostenintensiv.

### Aufgabe der Erfindung

In Kenntnis dieser bekannten Herstellungsverfahren ist es eine Aufgabe der vorliegenden Erfindung, ein für die Implantatherstellung geeignetes alternatives Homogenisierungsverfahren bereitzustellen, das kontinuierlich durchgeführt werden kann, keine Löse-/Ausfällungsschritte umfasst und frei von den oben erwähnten Nachteilen des Standes der Technik ist. Eine weitere Aufgabe der Erfindung ist es, ein alternatives Verfahren für die Implantatherstellung bereitzustellen.

### Kurze Beschreibung der Erfindung

Die Aufgabe der Erfindung wird durch das Bereitstellen eines Verfahrens zur Herstellung einer homogenen Pulvermischung aus mindestens einem Polymermaterial und mindestens einem aktiven Bestandteil gelöst, wobei das mindestens eine Polymermaterial und der mindestens eine aktive Bestandteil gemeinsam als Suspension in einem Dispersionsmedium homogenisiert werden, wobei sowohl das mindestens eine Polymermaterial als auch der mindestens eine aktive Bestandteil eine Löslichkeit im Dispersionsmedium aufweisen, die unter den Verfahrensbedingungen geringer ist als 1 g Substanz pro 1 Liter Dispersionsmedium, wobei das Polymermaterial ausgewählt wird aus der Gruppe, bestehend aus Polymeren und Copolymeren aus Milchsäure und/oder Glycolsäure, und der aktive Bestandteil einen Wirkstoff umfasst, der ausgewählt wird aus der Gruppe, bestehend aus Risperidon, Goserelin, Leuprorelin, Triptorelin, Octreotid und Anastrozol. Darüber hinaus stellt die Erfindung ein Verfahren zur Herstellung eines Implantates aus einer so hergestellten homogenen Pulvermischung bereit.

Insbesondere stellt die Erfindung die folgenden Mittel bereit.
(1) Ein Verfahren zur Herstellung einer homogenen Pulvermischung aus mindestens einem Polymermaterial und mindestens einem aktiven Bestandteil, wobei das mindestens eine Polymermaterial und der mindestens eine aktive Bestandteil gemeinsam als Suspension in einem Dispersionsmedium homogenisiert werden, wobei sowohl das mindestens eine Polymermaterial als auch der mindestens eine aktive Bestandteil eine Löslichkeit im Dispersionsmedium aufweisen, die unter den Verfahrensbedingungen geringer ist als 1 g Substanz pro 1 Liter Dispersionsmedium, wobei das Polymermaterial ausgewählt wird aus der Gruppe, bestehend aus Polymeren und Copolymeren aus Milchsäure und/oder Glycolsäure, und der aktive Bestandteil einen Wirkstoff umfasst, der ausgewählt wird aus der Gruppe, bestehend aus Risperidon, Goserelin, Leuprorelin, Triptorelin, Octreotid und Anastrozol.
(2) Ein Verfahren wie unter Punkt (1) beschrieben, in dem mechanisch homogenisiert wird.
(3) Ein Verfahren wie unter einem der Punkte (1) oder (2) beschrieben, in dem durch Mahlen homogenisiert wird.
(4) Ein Verfahren wie unter einem der Punkte (1) bis (3) beschrieben, in dem mit einer Dispergiervorrichtung vom Rotor-Stator-Typ homogenisiert wird, wodurch die festen Bestandteile der Suspension homogenisiert und gemahlen werden.
(5) Ein Verfahren wie unter einem der Punkte (1) bis (4) beschrieben, in dem kontinuierlich homogenisiert wird.
(6) Ein Verfahren wie unter einem der Punkte (1) bis (5) beschrieben, in dem das Polymermaterial aus der Gruppe ausgewählt wird, die aus Polylactiden und Polylactid-co-glycoliden besteht.
(7) Ein Verfahren wie unter einem der Punkte (1) bis (6) beschrieben, in dem das Dispersionsmedium einen Kohlenwasserstoff umfasst.
(8) Ein Verfahren wie unter einem der Punkte (1) bis (7) beschrieben, in dem das Dispersionsmedium Heptan umfasst.
(9) Ein Verfahren wie unter einem der Punkte (1) bis (8) beschrieben, in dem für 4 Minuten oder mehr homogenisiert wird.
(10) Ein Verfahren wie unter einem der Punkte (1) bis (9) beschrieben, das einen weiteren Schritt umfasst, in dem das Dispersionsmedium abgetrennt wird.
(11) Ein Verfahren wie unter Punkt (10) beschrieben, in dem das Dispersionsmedium durch Filtrieren abgetrennt, und die Pulvermischung danach gegebenenfalls getrocknet wird.
(12) Ein Verfahren wie unter Punkt (10) beschrieben, in dem das Dispersionsmedium durch Verdampfen abgetrennt wird.
(13) Ein Verfahren zur Herstellung eines Implantats, in dem eine homogene Pulvermischung nach einem Verfahren nach einem der Punkte (1) bis (12) hergestellt wird und die hergestellte homogene Pulvermischung zu einem Implantat weiterverarbeitet wird.
(14) Ein Verfahren zur Herstellung eines Implantats wie unter Punkt (13) beschrieben, in dem die homogene Pulvermischung extrudiert und zu Implantaten portioniert wird.

### Detaillierte Beschreibung der Erfindung

Die Aufgabe der Erfindung wird gelöst durch ein Verfahren zur Herstellung einer homogenen Pulvermischung aus mindestens einem Polymermaterial und mindestens einem aktiven Bestandteil, wobei das mindestens eine Polymermaterial und der mindestens eine aktive Bestandteil gemeinsam als Suspension in einem Dispersionsmedium homogenisiert werden, wobei sowohl das mindestens eine Polymermaterial als auch der mindestens eine aktive Bestandteil eine Löslichkeit im Dispersionsmedium aufweisen, die unter den Verfahrensbedingungen geringer ist als 1 g Substanz pro 1 Liter Dispersionsmedium, wobei das Polymermaterial ausgewählt wird aus der Gruppe, bestehend aus Polymeren und Copolymeren aus Milchsäure und/oder Glycolsäure, und der aktive Bestandteil einen Wirkstoff umfasst, der ausgewählt wird aus der Gruppe, bestehend aus Risperidon, Goserelin, Leuprorelin, Triptorelin, Octreotid und Anastrozol.

Die Erfinder der vorliegenden Erfindung haben überraschend gefunden, dass ein Implantat, das aus einer mit dem erfindungsgemäßen Verfahren hergestellten homogenen Pulvermischung hergestellt wird, ein außergewöhnlich vorteilhaftes Freisetzungsverhalten aufweist.

Durch das erfindungsgemäße Verfahren werden homogene Pulvermischungen aus mindestens einem Polymermaterial und mindestens einem aktiven Bestandteil hergestellt.

Der Begriff "Polymermaterial" wird im Sinne dieser Erfindung sowohl für einen polymeren Reinstoff (Polymer) als auch für eine Mischung aus mehreren Polymeren verwendet, jeweils optional unter Beimischung üblicher Zusatzstoffe. Der Begriff Polymer umfasst dabei isotaktische Polymere, Homopolymere und Copolymere, sowie Blockpolymere, Pfropfpolymere und dergleichen. Bevorzugt besitzt ein Polymer eine gewichtsmittlere Molmasse von 8.000 oder mehr, weiter bevorzugt von 10.000 bis 1.000.000.

Der Begriff "aktiver Bestandteil" wird im Sinne dieser Erfindung für jeden Bestandteil verwendet, der einer Pulvermischung bzw. dem daraus herzustellenden Implantat die gewünschte Wirkung verleihen kann, wie beispielsweise ein Wirkstoff, z.B. ein medizinischer Wirkstoff, ein Arzneimittel oder Medikament, oder ein Hormon oder ein Hormonantagonist bzw. -agonisten, ein Peptid-Wirkstoff, oder dergleichen. Alle diese Bestandteile und Wirkstoffe können als Reinstoffe, oder als übliche verträgliche Salze, Prodrugs oder dergleichen verwendet werden.

Erfindungsgemäß erfolgt die Homogenisierung des mindestens einen Polymermaterials und des mindestens einen aktiven Bestandteils in Suspension in einem Dispersionsmedium. "In Suspension in einem Dispersionsmedium" bedeutet im Sinne dieser Erfindung, dass sowohl Polymermaterial(ien) als auch aktive(r) Bestandteil(e) in dem Dispersionsmedium (z.T. auch Dispersionsmittel genannt) im wesentlichen unlöslich sind.

Eine Substanz wird als "im wesentlichen unlöslich" bezeichnet, wenn ihre Löslichkeit im Dispersionsmedium unter den Verfahrensbedingungen geringer ist als 1 g Substanz pro 1 Liter Dispersionsmedium, vorzugsweise geringer als 0,1 g Substanz pro 1 Liter Dispersionsmedium, und besonders bevorzugt geringer als 0,01 g Substanz pro 1 Liter Dispersionsmedium.

In alternativer Weise wird eine Substanz als "im wesentlichen unlöslich" bezeichnet, wenn ihr Löslichkeitsprodukt im Dispersionsmedium unter den Verfahrensbedingungen geringer ist als 1 x 10⁻⁸ mol/l, vorzugsweise geringer als 1 x 10⁻¹⁰ mol/l, und besonders bevorzugt geringer als 1 x 10⁻¹² mol/l.

Die Löslichkeit bzw. das Löslichkeitsprodukt von Polymermaterial(ien) und aktivem(n) Bestandteil(en) im jeweiligen Dispersionsmedium kann nach Standardverfahren bestimmt werden bzw. entsprechenden Tabellenwerken entnommen werden.

"Homogenisieren" bedeutet im Sinne dieser Erfindung, dass Polymermaterial(ien) und aktive(r) Bestandteil(e) homogen durchmischt werden. Als "homogen" bzw. "homogen durchmischt" wird insbesondere eine Pulvermischung bezeichnet, deren Zusammensetzung über die gesamte Pulvermischung gleich ist. Beispielsweise ist in einer erfindungsgemäß homogenen Pulvermischung aus einem Polymer und einem Wirkstoff die Konzentration des Wirkstoffes in der gesamten Pulvermischung gleich. Da eine Pulvermischung immer aus zwei oder mehreren unterschiedlichen festen Komponenten besteht, also Partikel unterschiedlicher Komponenten umfasst, wird die Homogenität nicht auf der Ebene der einzelnen Partikel bestimmt, sondern bezüglich eines vorgegebenen Volumens. Bevorzugt wird zur Definition ein Volumen von 5 mm³ herangezogen, weiter bevorzugt ein Volumen von 1 mm³. Besonders bevorzugt ist die Zusammensetzung einer erfindungsgemäßen homogenen Pulvermischung in jedem Kubikmillimeter davon gleich, weist also in jedem Kubikmillimeter dieselbe Wirkstoffkonzentration auf.

Alternativ zur Wirkstoffkonzentration pro Volumeneinheit kann auch die Konzentration des Wirkstoffes pro Gewichtseinheit der Pulvermischung angegeben werden, beispielsweise als Gehalt des Wirkstoffes in mg pro 100 mg der Pulvermischung (Gew.-%). Zur Bestimmung der Wirkstoffkonzentration pro Gewichtseinheit kann z.B. ein bestimmter Teil der Pulvermischung abgenommen werden (z.B. eine abgewogene Menge von 100 mg), und der Gehalt des Wirkstoffes darin bestimmt werden. Entsprechend weist eine homogene Pulvermischung in jeder Gewichtseinheit (z.B. 100 mg) die gleiche Wirkstoffkonzentration auf.

Der Gehalt des Wirkstoffes pro Volumeneinheit bzw. Gewichtseinheit kann beispielsweise durch Gaschromatographie oder HPLC (Hochleistungsflüssigchromatographie) mit UV-Detektion bestimmt werden.

Im Sinne dieser Erfindung wird von einer gleichen Wirkstoffkonzentration ausgegangen, wenn die für jedes beliebige Standardvolumen bzw. jede beliebige Gewichtseinheit der Pulvermischung bestimmte Konzentration des Wirkstoffes um weniger als 10% von der gemittelten Wirkstoffkonzentration in allen gemessenen Standardvolumen bzw. Gewichtseinheiten abweicht. Weiter bevorzugt beträgt die Abweichung der einzelnen Wirkstoffkonzentrationen von dem Mittelwert 5% oder weniger, noch weiter bevorzugt 3% oder weniger, und besonders bevorzugt 1% oder weniger.

Ein Mittelwert im Sinne dieser Erfindung ist ein arithmetischer Mittelwert (M), der erhalten wird, indem die Summe (Σ) aller Einzelwerte (xₙ) durch die Anzahl der Einzelwerte (n) dividiert wird (M = (Σ xₙ)/n). Die Abweichung (dₙ) eines Einzelwertes (xₙ) von dem Mittelwert (M) entspricht der Differenz zwischen Einzelwert und Mittelwert (dₙ = xₙ - M). Als ein weiteres Mass für die Abweichung kann auch die Standardabweichung (σ), die Stichprobenstandardabweichung (S), und/oder die relative Standardabweichung (% RSD) angegeben werden. Dabei wird die Standardabweichung (σ) als Quadratwurzel des Quotienten aus der Summe aller quadrierten Abweichungen der Einzelwerte vom Mittelwert und der Anzahl der Einzelwerte (n) berechnet (σ = (∑ dₙ²)/n)^{-1/2}), und die Stichprobenstandardabweichung (S) wird als Quadratwurzel des Quotienten aus der Summe aller quadrierten Abweichungen der Einzelwerte vom Mittelwert und der um Eins verringerten Anzahl der Einzelwerte (n - 1) berechnet (S = (∑ dₙ²)/(n-1))^{-1/2}). Die relative Standardabweichung (% RSD) wird als Produkt aus 100% und dem Quotienten aus Stichprobenstandardabweichung und Mittelwert berechnet (% RSD = 100% · S/M).

Bevorzugt werden die Teilchen des mindestens einen Polymermaterials und des mindestens einen aktiven Bestandteils bezüglich ihrer jeweiligen Partikelgröße und/oder Partikelform vereinheitlicht, so dass die hergestellte Pulvermischung aus gleich großen und/oder uniformen Partikeln besteht. Im Sinne dieser Erfindung werden Partikel als gleich groß angesehen, wenn ihre Größe in einem Bereich von 0 bis 30%, bevorzugt von 0 bis 10% von einer mittleren Partikelgröße abweichen.

Bevorzugt beträgt die mittlere Partikelgröße in der hergestellten homogenen Pulvermischung weniger als 500 µm, weiter bevorzugt von 1 bis 500 µm, weiter bevorzugt von 2 bis 400 µm, und besonders bevorzugt von 5 bis 300 µm.

Die mittlere Partikelgröße kann mit jedem geeigneten Verfahren bestimmt werden. Ein bevorzugtes Verfahren zur Bestimmung der mittleren Partikelgröße ist die Bestimmung der Partikelgröße durch Ausmessen in einer durch optische Mikroskopie erhaltenen Aufnahme. Dabei wird eine Probe bestimmten Volumens entnommen und bevorzugt lichtmikroskopisch untersucht. Die jeweils größte Dimension der einzelnen Partikel wird unabhängig von der jeweiligen Partikelform bestimmt. Dabei kann bevorzugt ein entsprechendes Bilderfassungs- und -auswertungs-system und/oder eine entsprechende Software verwendet werden. Die mittlere Partikelgröße wird bevorzugt bezogen auf die Gesamtanzahl der in der Probe enthaltenen Partikel angegeben. Aus statistischen Gründen werden vorzugsweise mehrere Proben unabhängig voneinander untersucht und dann ein Mittelwert gebildet. Bevorzugt werden mindestens zwei Proben untersucht, weiter bevorzugt mindestens drei, und besonders bevorzugt mindestens zehn Proben. Die Partikelgröße kann nach diesem Verfahren sowohl an getrockneten Partikeln gemessen werden, als auch an Partikeln, die in dem Dispersionsmedium dispergiert bzw. mit diesem benetzt sind. Sollte bei dispergierten Partikeln eine Agglomeratbildung beobachtet werden, wird die Bestimmung der Partikelgrößen bevorzugt im getrockneten Zustand durchgeführt.

Dadurch, dass im erfindungsgemäßen Verfahren in Suspension homogenisiert wird, umfasst das Verfahren keine Verfahrensschritte, in denen einer oder mehrere der Bestandteile gelöst und/oder ausgefällt werden. Entsprechend bleiben alle Bestandteile in ihrer ursprünglichen Kristallform und behalten ihre ursprünglichen Eigenschaften bei. Weiterhin erfolgt das Homogenisieren aller Bestandteile der hergestellten Pulvermischung vorzugsweise in einem einzigen Schritt, was nicht nur zeit-, energie- und kostensparend ist, sondern auch die Wahrscheinlichkeit einer Zersetzung eines oder mehrerer der Bestandteile verringert.

Bevorzugt wird im erfindungsgemäßen Verfahren mechanisch homogenisiert. Mechanisches Homogenisieren kann beispielsweise durch mechanisches Rühren erfolgen, vorzugsweise durch mechanisches Rühren unter Verwendung eines rotierenden Rührers mit hohen Drehgeschwindigkeiten. Mechanisches Homogenisieren kann beispielsweise durch mechanisches Zerkleinern erfolgen, z.B. durch Mahlen, Reiben, Rühren, Zerstoßen und dergleichen. Alternativ kann auch durch Ultraschall oder dergleichen homogenisiert werden.

Bevorzugt wird im erfindungsgemäßen Verfahren durch Mahlen homogenisiert. Es wurde gefunden, dass ein Homogenisieren durch Mahlen in Suspension (auch als Nassvermahlen bezeichnet) auch mit empfindlichen Bestandteilen, insbesondere empfindlichen Wirkstoffen, durchgeführt werden kann, die sich bei "trockenem" Mahlen zersetzen. Es wurde ferner gefunden, dass in Suspension auch Polymermaterialien gemahlen werden können, die "trocken" nicht gemahlen werden können, da sie dazu zu weich sind und/oder beim Mahlvorgang schmelzen, schmieren, oder dergleichen. Besonders bevorzugt wird unter Temperaturkontrolle gemahlen, wobei die Temperatur unterhalb der Glasübergangstemperatur des Polymers gehalten wird. Besonders bevorzugt wird bei einer Temperatur von weniger als 35°C, besonders bevorzugt von weniger als 30°C und am meisten bevorzugt bei 22 bis 25°C gemahlen. Dazu kann mit üblichen Verfahren gekühlt werden. Beispielsweise können Polylactide oder Polylactid-co-Glycolide in Suspension kontrolliert unterhalb ihrer jeweiligen Glasübergangstemperatur gemahlen werden. Die Glasübergangstemperatur bedeutet dabei die Temperatur, bei der ganz oder teilweise amorphe Polymere von dem hochviskosen, flexiblen Zustand in den glasartigen oder spröden Zustand übergehen. Eine Messung der Glasübergangstemperatur kann beispielsweise mit Hilfe der dynamischen Differenzkalorimetrie (DSC) erfolgen.

Bevorzugt wird im erfindungsgemäßen Verfahren mit einer Dispergiervorrichtung vom Rotor-Stator-Typ, vorzugsweise mit hoher Drehzahl des Rotors, homogenisiert. Dadurch werden die festen Bestandteile der Suspension homogen gerührt und gemahlen.

Bevorzugt beträgt die Drehzahl des Rotors 8000 UpM (Umdrehungen pro Minute) oder mehr, weiter bevorzugt 10000 UpM oder mehr, weiter bevorzugt 13000 UpM oder mehr, und besonders bevorzugt etwa 13500 UpM. Mit einer mittleren Drehzahl von 13500 UpM wurden besonders vorteilhafte Resultate erreicht.

Bevorzugt erfolgt der Homogenisierungsschritt für 4 bis zu 10 Minuten, bevorzugt bis zu 6 Minuten und besonders bevorzugt von 4 bis 5 Minuten. Nach Ablauf dieser Zeitspanne wurden in den Beispielen keine weiteren Verbesserungen der Eigenschaften der homogenen Pulvermischung beobachtet.

Eine "Dispergiervorrichtung vom Rotor-Stator-Typ" im Sinne der Erfindung ist insbesondere eine Dispergiervorrichtung mit einem geschlitzten zylinderförmigen Rotor, der sich mit hoher Drehzahl in einem geschlitzten zylinderförmigen Stator dreht, wodurch festes Material im bearbeiteten Medium sehr hohen Scher- und Schubkräften unterworfen und so zerkleinert (gemahlen) wird. Weiterhin treten im Zwischenraum zwischen Rotor und Stator (Scherspalt) zusätzlich große Turbulenzen auf, die zu einer optimalen Durchmischung der Suspension führen.

Bevorzugt beträgt die Umfangsgeschwindigkeit der Rotor-Stator-Anordnung von 10 bis 24 m/s.

Zur Ausführung der Erfindung geeignete Dispergiervorrichtungen sind kommerziell erhältlich. Beispielsweise kann eine von der Fa. IKA Werke GmbH hergestellte und unter der Bezeichnung "ULTRA-TURRAX Workstation" vertriebene Dispergiervorrichtung verwendet werden.

Bevorzugt wird im erfindungsgemäßen Verfahren kontinuierlich homogenisiert. Für eine kontinuierliche Homogenisierung kann beispielsweise ein Durchflusshomogenisator verwendet werden. Durch kontinuierlichen Betrieb können große Chargen der homogenen Pulvermischung in einem Arbeitsschritt hergestellt werden.

Bei dem erfindungsgemäßen Verfahren wird das mindestens eine Polymermaterial aus der Gruppe ausgewählt, die aus Polymeren und Copolymeren aus Milchsäure und/oder Glycolsäure besteht, insbesondere Polylactide und Polylactid-co-glycolide.

Bei dem erfindungsgemäßen Verfahren umfasst der aktive Bestandteil einen Wirkstoff, der ausgewählt wird aus der Gruppe, bestehend aus Risperidon, Goserelin, Leuprorelin, Triptorelin, Octreotid und Anastrozol.

Das Dispersionsmedium kann ein Reinstoff oder eine Mischung aus mehreren Stoffen sein. Besonders bevorzugt wird im erfindungsgemäßen Verfahren das Dispersionsmedium (Dispersionsmittel) ausgewählt aus der Gruppe der Kohlenwasserstoffe, insbesondere der gesättigten Kohlenwasserstoffe. Besonders bevorzugt umfasst das Dispersionsmedium ein Alkan mit vier bis zehn Kohlenstoffatomen, weiter bevorzugt mit sechs bis acht Kohlenstoffatomen. Dabei kann das Dispersionsmedium entweder ein reines Alkan sein, oder eine Mischung aus mehreren Alkanen und/oder mehreren isomeren Alkanen. Besonders bevorzugt umfasst das Dispersionsmedium Hexan oder Heptan, insbesondere Heptan. Besonders bevorzugt ist das Dispersionsmedium n-Heptan. Gemäß einer bevorzugten Ausführungsform wird ein trockenes Dispersionsmedium, insbesondere trockenes Heptan eingesetzt.

Bevorzugt umfasst das erfindungsgemäße Verfahren einen weiteren Schritt, in dem das Dispersionsmedium abgetrennt wird. So kann die homogene Pulvermischung im trockenen Zustand, d.h. ohne Dispersionsmittel, erhalten werden, was bei der Weiterverarbeitung zu einem Implantat vorteilhaft ist.

Bevorzugt wird im erfindungsgemäßen Verfahren das Dispersionsmittel durch Filtrieren abgetrennt. Eine Abtrennung durch Filtrieren erfordert keine Erhöhung der Temperatur, wie z.B. ein Verdampfen des Dispersionsmittels, so dass die Bestandteile der Pulvermischung keiner thermischen Belastung ausgesetzt werden müssen.

Optional kann auch ein Trockenschritt nach Abtrennen des Dispersionsmittels durchgeführt werden, in dem Dispersionsmittelreste, die nach dem Filtrieren in der Pulvermischung zurückbleiben, entfernt werden, gegebenenfalls unter erhöhter Temperatur und/oder vermindertem Druck und/oder unter Verwendung eines Gasstroms, bevorzugt eines Stroms aus Luft oder einem Inertgas, wie z.B. Stickstoff, Argon oder dergleichen. Bevorzugt ist auch die Verwendung einer Gefriertrocknungsvorrichtung zum Trocknen der Pulvermischung.

Bevorzugt wird im erfindungsgemäßen Verfahren das Dispersionsmittel durch Verdampfen abgetrennt. Eine Abtrennung des Dispersionsmittels durch Verdampfen kann bei unempfindlichen Polymermaterialien und/oder Wirkstoffen vorteilhaft sein. Bei empfindlichen Wirkstoffen und/oder Polymermaterialien kann gegebenenfalls unter verringertem Druck verdampft werden.

Weiter wird die Aufgabe der Erfindung gelöst durch die Bereitstellung eines Verfahrens zur Herstellung eines Implantats, in dem eine durch ein zuvor beschriebenes erfindungsgemäßes Verfahren hergestellte homogene Pulvermischung zu einem Implantat weiterverarbeitet wird. Die Weiterverarbeitung einer Pulvermischung zu einem Implantat kann beispielsweise durch Pressen oder Extrudieren und Portionieren erfolgen und wird beispielsweise im eingangs zitierten Stand der Technik beschrieben.

Bevorzugt wird im erfindungsgemäßen Verfahren die homogene Pulvermischung extrudiert und zu Implantaten portioniert. Dieses Verfahren der Implantatherstellung wird z.B. in WO 98/09613 beschrieben.

Im Sinne dieser Erfindung ist ein Implantat ein übliches Implantat oder ein implantierbares Arzneimitteldepot, welches in der Arzneimitteltherapie, z.B. bei der Langzeitbehandlung von Erkrankungen, eingesetzt werden kann. Ein Implantat umfasst einen aktiven Bestandteil, wie beispielsweise ein Arzneimittel (Medikament bzw. Wirkstoff).

Ein Implantat kann jede übliche Form aufweisen. Geeignete Formen des Implantats sind zylindrisch oder dergleichen. Vorzugsweise ist ein Implantat in Form eines zylindrischen Stäbchens aus der Polymer/Wirkstoff-Matrix ausgebildet. Ein derartiges Implantat kann bevorzugt durch Extrusion hergestellt werden. Alternativ kann ein Implantat kugelförmig sein, z.B. in Form eines Pellets, oder dergleichen.

Bevorzugte Abmessungen eines Implantats sind eine Länge von 5 bis 25 mm, weiter bevorzugt von 10 bis 20 mm, und ein Querschnitt von 0,8 bis 2,5 mm, weiter bevorzugt 1,1 bis 1,6 mm. Bevorzugt umfasst ein Implantat von 0,1 bis 50 Gewichts-%, weiter bevorzugt von 5 bis 40 Gew.-%, und besonders bevorzugt von 15 bis 30 Gew.-%, Wirkstoff und von 50 bis 99,9 Gew.-%, weiter bevorzugt von 60 bis 95 Gew.-%, und besonders bevorzugt von 70 bis 85 Gew.-%, biologisch abbaubares Polymermaterial. Bevorzugt beträgt die Gesamtmasse des Implantats von 10 bis 100 mg, weiter bevorzugt von 15 bis 75 mg, und besonders bevorzugt von 20 bis 50 mg.

In einer besonders bevorzugten Ausführungsform umfasst ein stäbchenförmiges Implantat mit Abmessungen von 12 bis 16 mm Länge und von 1,05 bis 1,25 mm Durchmesser 3,5 bis 3,8 mg Goserelin und 15 mg bis 25 mg biologisch abbaubares Polymermaterial, bevorzugt Polylactid-co-glycolid. Dieses Implantat ist bevorzugt als 1-Monatsformulierung geeignet. In einer anderen besonders bevorzugten Ausführungsform umfasst ein stäbchenförmiges Implantat mit Abmessungen von 17 bis 25 mm Länge und von 1,40 bis 1,60 mm Durchmesser 10,6 bis 11,0 mg Goserelin und 35 mg bis 55 mg biologisch abbaubares Polymermaterial, bevorzugt Polylactid-co-glycolid. Dieses Implantat ist bevorzugt als 3-Monatsformulierung geeignet. Diese Implantate können auch alternative bzw. zusätzliche Wirkstoffe umfassen, wie z.B. Leuprorelin und/oder Octreotid, wobei die jeweiligen Dosierungen entsprechend angepasst werden können.

### Beispiele

In den folgenden Beispielen wird die Erfindung anhand der Herstellung eines Implantats mit dem Wirkstoff Goserelin detaillierter beschrieben. Als Polymermaterialien wurden verwendet: ein Polylactid mit einer Glasübergangstemperatur von 44 bis 48°C, das unter dem Handelsnamen Resomer R 202H von der Firma Evonik Röhm GmbH bezogen wurde, und ein Polylactid-co-glycolid mit einer Glasübergangstemperatur von 42 bis 46°C, das unter dem Handelsnamen Resomer RG 502H von der Firma Evonik Röhm GmbH bezogen wurde. Als Dispergiervorrichtung wurde ein Modell verwendet, das von der Fa. IKA Werke GmbH hergestellt und unter der Bezeichnung ULTRA-TURRAX Workstation vertrieben wird. Als Extruder wurde ein Göttfert Rheograph 25E verwendet. Die vorliegende Erfindung ist aber nicht auf die in diesen Beispielen verwendeten Substanzen oder Vorrichtungen eingeschränkt.

### Beispiel 1: Herstellung einer homogenen Pulvermischung

Es wurde eine trockene Mischung aus 25% (Gewichts-%, auch im folgenden, sofern nicht anders angegeben) Wirkstoff (Goserelin), 67,5% Polylactid und 7,5% Polylactid-co-glycolid hergestellt. 4,5 g der vorbereiteten Mischung wurden in einem Glasgefäß mit 30 ml Heptan als Dispersionsmedium versetzt und unter Temperaturkontrolle für insgesamt vier Minuten dispergiert. Die Temperatur stieg im Verlauf der Dispergierung von 25,0°C auf 31,7°C an (s. Tabelle 1). Damit wurde die Glasübergangstemperatur der eingesetzten Polymermaterialien zu keiner Zeit überschritten.

**Tabelle 1: Temperaturverlauf der Dispergierung**

| Dispergierdauer [Min.] | Temperatur Dispersion [°C] |
|---|---|
| 1 | 25,0 |
| 2 | 27,5 |
| 3 | 30,8 |
| 4 | 31,7 |

Der Fortschritt der Homogenisierung der Mischung während der Dispergierung wurde durch periodische Probenentnahme überprüft. Dazu wurden jeweils drei unabhängig voneinander entnommene Probenvolumen durch Filtrieren über eine Glasfilternutsche vom Dispersionsmedium Heptan getrennt, für eine Stunde bei einem Zielvakuum von 0,3 mbar in einer Gefriertrocknungsanlage getrocknet und hinsichtlich ihrer Zusammensetzung untersucht. Der Restgehalt an Heptan betrug in den so behandelten Proben von 4986 bis 6415 ppm. Es wurde festgestellt, dass eine homogene Pulvermischung mit einem angestrebten Wirkstoffgehalt nach 4 Minuten erhalten werden kann. Eine lichtmikroskopische Untersuchung ergab, dass die einzelnen Partikel der homogenisierten Pulvermischung eine maximale Partikelgröße von 0,02 mm bis 0,2 mm hatten.

### Beispiel 2: Herstellung von Implantaten

Aus den in Beispiel 1 hergestellten homogenen Pulvermischungen wurden bei 83°C Extrusionstemperatur mit einer Geschwindigkeit von 0,008 mm/s zu Implantaten extrudiert. Dabei kann ein Heptangehalt von kleiner 5000 ppm durch Wahl der Temperatur im Bereich von 45 bis 65°C in der Vorkompressionsphase gewährleistet werden. Eine an den Implantaten durchgeführte Reinheitsuntersuchung zeigte, dass diese mit einem Gesamtgehalt an Verunreinigungen von 2,5% oder weniger den Anforderungen einer Freigabespezifikation entsprachen. Eine Untersuchung des Freisetzungsverhaltens ergab ein gewünschtes Freisetzungsprofil.

### Beispiel 3: Prüfung der Homogenität

Zur Bestimmung der Homogenität der hergestellten Pulvermischung wurde Beispiel 1 wiederholt, wobei der Dispersion aus Feststoffen in Heptan zu bestimmten Zeitpunkten jeweils drei Proben entnommen wurden. Eine Probenentnahme erfolgte jeweils nach einer halben Minute, nach einer Minute, sowie nach zwei, drei, vier, fünf und zehn Minuten. Dabei wurden jeweils gleiche Volumina der Dispersion mit einer Pipette entnommen, das Dispergiermittel Heptan wurde entfernt, und die Wirkstoffkonzentration im verbleibenden Feststoff (Sollprobenmenge: 27 bis 44 mg) wurde mittels Gaschromatographie bestimmt. Zur besseren Vergleichbarkeit wurden die Werte auf 100 mg Pulvermischung normiert. Die Ergebnisse sind in Tabelle 2 zusammengestellt.

**Tabelle 2: Homogenitätsprüfung**

| Dispergierdauer | Probennr. | Matrixkonz. [%] | MW Matrixkonz. [mg/100mg] | Abw. in % vom MW Matrixkonz. | RSD[%] |
|---|---|---|---|---|---|
| 0,5min | 1* | 7,73 | 17,13 | -55,1 | 47,83 |
| | 2 | 21,39 | | 24,2 | |
| | 3* | 22,52 | | 30,8 | |
| 1min | 1* | 13,14 | 19,86 | -33,8 | 29,51 |
| | 2 | 22,53 | | 13,4 | |
| | 3 | 23,92 | | 20,4 | |
| 2min | 1 | 20,13 | 22,31 | -9,8 | 8,46 |
| | 2 | 23,30 | | 4,5 | |
| | 3* | 23,49 | | 5,3 | |
| 3min | 1 | 21,21 | 22,95 | -7,6 | 8,27 |
| | 2 | 22,67 | | -1,2 | |
| | 3 | 24,97 | | 8,8 | |
| 4min | 1 | 23,12 | 23,32 | -0,9 | 0,77 |
| | 2 | 23,46 | | 0,6 | |
| | 3 | 23,39 | | 0,3 | |
| 5min | 1 | 23,24 | 23,28 | -0,2 | 0,16 |
| | 2 | 23,31 | | 0,1 | |
| | 3 | 23,29 | | 0,0 | |
| 10min | 1* | 23,24 | 23,37 | -0,6 | 0,5 |
| | 2* | 23,46 | | 0,4 | |
| | 3 | 23,41 | | 0,2 | |

| | | | | | |
|---|---|---|---|---|---|
| (* - die entnommene Probenmenge betrug weniger als 27 mg Probe) | | | | | |

Aus den in Tabelle 2 gezeigten Ergebnissen ist klar ersichtlich, dass die Abweichung der Einzelgehälter vom Mittelwert der Matrixkonzentration mit zunehmender Dispergierdauer geringer wurde, d.h. die Homogenität der Probe stieg. Ab einer Dispergierdauer von 4 Minuten liegen alle Einzelgehälter unterhalb einer Abweichung von 2,5%. Die relative Standardabweichung (% RSD) betrug in diesen Proben weniger als 1%.

### Beispiel 4: Bestimmung von Partikelgrössen

Zur Untersuchung des Einflusses des erfindungsgemäßen Verfahrens auf die Partikelgröße von Polymer und Wirkstoff wurden die Partikelgrößen der Ausgangsstoffe und der hergestellten Pulvermischung mit Hilfe der Stereomikroskopie mit graphischer Auswertung bestimmt. Die Proben wurden hierzu in Silikonöl präpariert und im Durchlicht betrachtet.

Zur Messung der Partikelgröße wurde ein Stereomikroskop (Leica; Geräte-Nr. 2324) verwendet, das mit Durchlicht und einer digitalen Kamera (Color View, Olympus; Geräte-Nr. 1469-1479) ausgestattet war. Zur Vergrößerung wurde ein 10fach Okular verwendet. Die Bilder wurden mit der Software Analysis Doku 5 (Olympus) erstellt. Mittels dieser Software wurde auch die Messung der Partikelgrößen durchgeführt. Die tägliche Kalibrierung der Längenmessung erfolgte über die Vermessung eines Okularmikrometers (Carl Zeiss, Maßstab 5 + 100/100 mm; Geräte-Nr. E_0081). Die weitere Auswertung erfolgte mittels der Software MS Excel.

Zur Probenaufbereitung wurde jeweils eine Spatelspitze des zu untersuchenden Ausgangstoffs bzw. der zu untersuchenden Polymer-Wirkstoff-Mischung in ein 1,5 ml Eppendorf-Cup gegeben und mit ca. 0,5 ml Silikonöl versetzt. Unter leichtem Rühren wurde der Ansatz in eine gleichmäßig trübe Probe überführt. Hierbei war zu beobachten, dass vorhandene Agglomerate sich trennten. Ein Tropfen jeder Probe wurde auf einen Objektträger übertragen und direkt vermessen. Dazu wurde etwa von der Mitte des Probentropfens eine Aufnahme bei der voreingestellten Vergrößerung gemacht und das digitale Bild zur weiteren Auswertung per Software gespeichert.

Zur Vermessung der Partikelgrößen wurde für jede Probe die Aufnahme in 12 (3 x 4) gleichgroße Felder aufgeteilt. Dabei wurde das Messwerkzeug der Software immer an der jeweils größten Dimension des Partikels angelegt. Die Messung wurde jeweils an drei separat aufgearbeiteten Proben durchgeführt. Die gemessenen Werte wurden auf die tatsächliche Länge mit Hilfe des mit dem Okularmikrometer berechneten Faktor umgerechnet. Der Mittelwert, die relative Standardabweichung, das Minimum und das Maximum aus allen Messungen zu jeder Probe wurden berechnet.

Allgemein wurde beobachtet, dass die Teilchen des Wirkstoffs Goserelinacetat eine plättchenförmige Struktur haben und eine sehr breite Partikelgrößenverteilung zeigen. Das Polymer Resomer RG 502H weist neben größeren Partikeln viele kleine, teilweise fadenförmige Strukturen auf. Nach Homogenisierung wurde eine deutliche Reduzierung der Partikelgröße der plättchenförmigen Wirkstoffpartikel beobachtet. Gleichzeitig wurden weniger fadenförmige Polymerpartikel beobachtet.

Als erfindungsgemäßes Beispiel wurden 20,5% Goserelinacetat und 79,5% Resomer RG 502H zusammengegeben, um eine Vormischung herzustellen. 3 g dieser Vormischung wurden in 50 ml Heptan bei 11000 UpM für 4 x 1 Minuten mit einer Zwischenkühlzeit von 3 Minuten homogenisiert. Nach Filtration über eine Glasfritte (Wasserstrahlpumpe) wurde der feste Rückstand für 24 Stunden bei 280 mbar getrocknet.

Als Vergleichsbeispiele wurden zwei identisch zusammengesetzte Proben mittels Kryovermahlung (flüssiger Stickstoff, -196°C) homogenisiert. Dabei wurden jeweils 15 Zyklen mit einer Rate von 10/s (Vergleichsbeispiel A) bzw. 15/s (Vergleichsbeispiel B), jeweils mit einer Zwischenkühlzeit von 1 Minute, durchgeführt.

Die Ergebnisse der Partikelgrößenmessungen sind in Tabelle 3 zusammengefasst.

**Tabelle 3: Partikelgrößen (n = 3)¹**

| **Material (Mischverfahren)** | **Mittelwert / µm** | **rel. Standard abweichung / %** | **Minimum/ µm** | **Maximum/ µm** |
|---|---|---|---|---|
| Wirkstoff: Goserelinacetat | 42,53 | 16 | 7,79 | 244,47 |
| Polymer: Resomer RG 502H | 40,04 | 14 | 12,19 | 153,39 |
| Polymer-Wirkstoff-Mischung (Vergleichsbeispiel A) | 26,73 | 17 | 8,62 | 93,57 |
| Polymer-Wirkstoff-Mischung (Vergleichsbeispiel B) | 28,25 | 6 | 9,29 | 81,94 |
| Polymer-Wirkstoff-Mischung (Beispiel, erfindungsgemäß) | 34,92 | 14 | 10,91 | 110,97 |

| | | | | |
|---|---|---|---|---|
| ¹Vergleichsbeispiel A: n= 2; je Probenaufarbeitung wurden ca. 60 Partikel vermessen | | | | |

Die Daten in Tabelle 3 zeigen, dass beide Homogenisierungsverfahren zu einer Verkleinerung der Partikelgröße der Ausgangsstoffe führen. Darüber hinaus ist die Partikelgrößenverteilung in den Polymer-Wirkstoff-Mischungen enger als in den Ausgangsstoffen selbst.

Die Daten der einzelnen Pulvermischungen sind dabei sehr gut untereinander vergleichbar. Gegenüber den Ausgangstoffen sind jedoch Veränderungen messbar. Die Polymer-Wirkstoff-Mischungen zeigen mit 26,73 bis 34,92 µm eine deutliche Verkleinerung des Mittelwerts gegenüber dem Wirkstoff (42,53 µm) und dem Polymer (40,04 µm). Auch die maximal in den Pulvermischungen gemessenen Partikelgrößen (81,94 bis 110,97 µm) liegen deutlich unterhalb der für die Ausgangsstoffe gemessenen Maxima (244,47 und 153,39). Die Partikelgrößenverteilung ist in allen Proben vergleichbar. Lediglich Vergleichsbeispiel B, das im Vergleich zu Vergleichsbeispiel A mit erhöhter Mahlrate vermahlen wurde, zeigt eine engere Verteilung der Partikel, ausgedrückt als relative Standardabweichung. In beiden Verfahren werden die Partikel zerkleinert (gemahlen).

### Industrielle Anwendbarkeit

Mit dem Verfahren der vorliegenden Erfindung wird eine homogene Pulvermischung hergestellt, die zu einem Implantat mit vorteilhaften Eigenschaften weiterverarbeitet werden kann.

## Patentansprüche

1. Verfahren zur Herstellung einer homogenen Pulvermischung aus mindestens einem Polymermaterial und mindestens einem aktiven Bestandteil, wobei das mindestens eine Polymermaterial und der mindestens eine aktive Bestandteil gemeinsam als Suspension in einem Dispersionsmedium homogenisiert werden, wobei sowohl das mindestens eine Polymermaterial als auch der mindestens eine aktive Bestandteil eine Löslichkeit im Dispersionsmedium aufweisen, die unter den Verfahrensbedingungen geringer ist als 1 g Substanz pro 1 Liter Dispersionsmedium,
**dadurch gekennzeichnet,**
**dass** das Polymermaterial ausgewählt wird aus der Gruppe, bestehend aus Polymeren und Copolymeren aus Milchsäure und/oder Glycolsäure, und
**dass** der aktive Bestandteil einen Wirkstoff umfasst, der ausgewählt wird aus der Gruppe, bestehend aus Risperidon, Goserelin, Leuprorelin, Triptorelin, Octreotid und Anastrozol.

2. Verfahren nach Anspruch 1, **dadurch gekennzeichnet, dass** mechanisch homogenisiert wird.

3. Verfahren nach Anspruch 1 oder 2, **dadurch gekennzeichnet, dass** durch Mahlen homogenisiert wird.

4. Verfahren nach einem der vorherigen Ansprüche, **dadurch gekennzeichnet, dass** mit einer Dispergiervorrichtung vom Rotor-Stator-Typ homogenisiert wird, wodurch die festen Bestandteile der Suspension homogenisiert und gemahlen werden.

5. Verfahren nach einem der vorherigen Ansprüche, **dadurch gekennzeichnet, dass** kontinuierlich homogenisiert wird.

6. Verfahren nach einem der vorherigen Ansprüche, **dadurch gekennzeichnet, dass** das Polymermaterial ausgewählt wird aus der Gruppe, bestehend aus Polylactiden und Polylactid-co-glycoliden.

7. Verfahren nach einem der vorherigen Ansprüche, **dadurch gekennzeichnet, dass** das Dispersionsmedium einen Kohlenwasserstoff umfasst.

8. Verfahren nach einem der vorherigen Ansprüche, **dadurch gekennzeichnet, dass** das Dispersionsmedium Heptan umfasst.

9. Verfahren nach einem der vorherigen Ansprüche, **dadurch gekennzeichnet, dass** für 4 Minuten oder mehr homogenisiert wird.

10. Verfahren nach einem der vorherigen Ansprüche, umfassend einen weiteren Schritt, in dem das Dispersionsmedium abgetrennt wird.

11. Verfahren nach Anspruch 10, **dadurch gekennzeichnet, dass** das Dispersionsmedium durch Filtrieren abgetrennt, und die Pulvermischung danach gegebenenfalls getrocknet wird.

12. Verfahren nach Anspruch 10, **dadurch gekennzeichnet, dass** das Dispersionsmedium durch Verdampfen abgetrennt wird.

13. Verfahren zur Herstellung eines Implantats, **dadurch gekennzeichnet, dass** eine homogene Pulvermischung nach einem Verfahren nach einem der vorherigen Ansprüche hergestellt wird; und
die hergestellte homogene Pulvermischung zu einem Implantat weiterverarbeitet wird.

14. Verfahren nach Anspruch 13, **dadurch gekennzeichnet, dass** die homogene Pulvermischung extrudiert und zu Implantaten portioniert wird.

## Claims

1. A process for producing a homogeneous powder mixture of at least one polymer material and at least one active ingredient, wherein the at least one polymer material and the at least one active ingredient are homogenized together as a suspension in a dispersion medium,
wherein both the at least one polymer material as well as the at least one active ingredient show a solubility in the dispersion medium, which is lower than 1 g substance per 1 liter of dispersion medium under the process conditions,
**characterized in that**
the polymer material is selected from the group consisting of polymers and copolymers of lactic acid and/or glycolic acid, and that the active ingredient comprises an active agent selected from the group consisting of risperidone, goserelin, leuprorelin, triptorelin, octreotide and anastrozole.

2. A process according to claim 1, **characterized in that** homogenization is carried out mechanically.

3. A process according to claim 1 or 2, **characterized in that** homogenization is carried out by grinding.

4. A process according to one of the preceding claims,
**characterized in that** homogenization is carried out with a dispersing device of the rotor-stator-type, whereby the solid components of the suspension are homogenized and ground.

5. A process according to one of the preceding claims,
**characterized in that** homogenization is carried out continuously.

6. A process according to one of the preceding claims,
**characterized in that** the polymer material is selected from the group consisting of polylactides and polylactide-co-glycolides.

7. A process according to one of the preceding claims,
**characterized in that** the dispersion medium comprises a hydrocarbon.

8. A process according to one of the preceding claims,
**characterized in that** the dispersion medium comprises heptane.

9. A process according to one of the preceding claims,
**characterized in that** homogenization is carried out for 4 minutes or more.

10. A process according to one of the preceding claims, comprising a further step in which the dispersion medium is separated.

11. A process according to claim 10, **characterized in that** the dispersion medium is separated by filtration, and the powder mixture is then optionally dried.

12. A process according to claim 10, **characterized in that** the dispersion medium is separated by evaporation.

13. A process for producing an implant, **characterized in that** a homogeneous powder mixture is produced by a process according to one of the preceding claims; and the produced homogenous powder mixture is further processed into an implant.

14. A process according to claim 13, **characterized in that** the homogeneous powder mixture is extruded and portioned to implants.

## Revendications

1. Procédé de préparation d'un mélange pulvérulent homogène à partir d'au moins un matériau polymère et d'au moins un ingrédient actif, dans lequel ledit au moins un matériau polymère et ledit au moins un ingrédient actif sont homogénéisés ensemble en suspension dans un milieu de dispersion, aussi bien ledit au moins un matériau polymère que ledit au moins un ingrédient actif présentent une solubilité dans le milieu de dispersion qui est inférieure à 1 g de substance pour 1 litre de milieu de dispersion, dans les conditions du procédé,
**caractérisé en ce que**
le matériau polymère est choisi dans le groupe constitué de polymères et de copolymères d'acide lactique et/ou d'acide glycolique, et
**en ce que** l'ingrédient actif comprend un principe actif choisi dans le groupe constitué de rispéridone, de goséréline, de leuproréline, de triptoréline, d'octreoïde et d'anastrozole.

2. Procédé selon la revendication 1, **caractérisé en ce que** l'homogénéisation s'effectue par voie mécanique.

3. Procédé selon la revendication 1 ou 2, **caractérisé en ce que** l'homogénéisation s'effectue par broyage.

4. Procédé selon l'une des revendications précédentes, **caractérisé en ce que** l'homogénéisation s'effectue par un dispositif de dispersion du type à rotor et à stator, moyennant quoi les constituants solides de la suspension sont homogénéisés et broyées.

5. Procédé selon l'une des revendications précédentes, **caractérisé en ce que** l'homogénéisation s'effectue en continu.

6. Procédé selon l'une des revendications précédentes, **caractérisé en ce que** le matériau polymère est choisi parmi le groupe constitué de polylactides et de polylactid-co-glycolides.

7. Procédé selon l'une des revendications précédentes, **caractérisé en ce que** le milieu de dispersion comprend un hydrocarbure.

8. Procédé selon l'une des revendications précédentes, **caractérisé en ce que** le milieu de dispersion comprend de l'heptane.

9. Procédé selon l'une des revendications précédentes, **caractérisé en ce que** l'homogénéisation s'effectue pendant 4 minutes ou plus.

10. Procédé selon l'une des revendications précédentes, comprenant une autre étape consistant à séparer le milieu de dispersion.

11. Procédé selon la revendication 10, **caractérisé en ce que** le milieu de dispersion est séparé par filtrage et le cas échéant le mélange pulvérulent est ensuite séché.

12. Procédé selon la revendication 10, **caractérisé en ce que** le milieu de dispersion est séparé par évaporation.

13. Procédé de réalisation d'un implant, **caractérisé en ce que** l'on prépare un mélange pulvérulent homogène par un procédé selon l'une des revendications précédentes, et
le mélange pulvérulent homogène préparé est transformé en un implant.

14. Procédé selon la revendication 13, **caractérisé en ce que** le mélange pulvérulent homogène est extrudé et mis en portions pour donner des implants.
